# EUROPEAN PATENT SPECIFICATION

(11) **EP 2 646 873 B1**
(45) Date of publication and mention of the grant of the patent: **24.08.2016**
(21) Application number: 11806013.6
(22) Date of filing: 30.11.2011
(51) Int. Cl.: G02F 1/361

(54) **STABLE FREE RADICAL CHROMOPHORES AND MIXTURES THEREOF, PROCESSES FOR PREPARING THE SAME, NONLINEAR OPTIC MATERIALS, AND USES THEREOF IN NONLINEAR OPTICAL APPLICATIONS**
STABILE FREIE RADIKALE CHROMOPHOREN UND MISCHUNGEN DARAUS, VERFAHREN ZU IHRER HERSTELLUNG, NICHTLINEARE OPTISCHE MATERIALIEN UND IHRE VERWENDUNG FÜR NICHTLINEARE OPTISCHE ANWENDUNGEN
CHROMOPHORES À RADICAL LIBRE STABLE ET LEURS MÉLANGES, PROCÉDÉS DE PRÉPARATION ASSOCIÉS, MATÉRIAUX OPTIQUES NON LINÉAIRES, ET LEURS UTILISATIONS DANS DES APPLICATIONS OPTIQUES NON LINÉAIRES

(30) Priority: 30.11.2010 US 418136 P
(43) Date of publication of application: 09.10.2013
(73) Proprietor: Lightwave Logic, Inc., Newark, DE 19711 (US)
(72) Inventor: GOETZ, Frederick, J., Newark DE 19710 (US); ASHTON, Andrew, Newark DE 19711 (US); GOETZ, Frederick, J., Wilmington DE 19810 (US); EATON, David, F., Wilmington DE 19805 (US); ARDUENGO, Anthony, J., Coaling AL 35449 (US); SIMMONS, Howard, E., Wilmington DE 19807 (US); RUNYON, Jason, W., Villa Rica GA 30180 (US)
(74) Representative: Kruspig, Volkmar
(86) International application number: PCT/US2011/062627
(87) International publication number: WO 2012/075130

(56) References cited:
- US-A1- 2008 139 812
- Third-Order Nanotechnologies: "Third-Generation Electro-Optic Plastics", , 9 January 2007 (2007-01-09), pages 1-12, XP002674009, Retrieved from the Internet: URL:http://third-order.com/print/third-ord er_tech3.pdf [retrieved on 2012-04-13]
- Lightwave Logic: "Lightwave Logic Files Sixth Patent Application", Press Release , 9 December 2009 (2009-12-09), XP002674010, Retrieved from the Internet: URL:http://www.lightwavelogic.com/pdf/LWLG _Patent_Press_Release_%20FINAL%2012-8-09.p df [retrieved on 2012-04-13]
- JEAN-FRANÇOIS NICOUD ET AL: "Hyperpolarizability of new nitro-aromatic-N-oxide species: nonlinear optical properties of a ferromagnetic organic material", CHEMICAL PHYSICS LETTERS, vol. 175, no. 3, 1 December 1990 (1990-12-01), pages 257-261, XP055024449, ISSN: 0009-2614, DOI: 10.1016/0009-2614(90)85552-N
- SANTO DI BELLA ET AL: "Large Second-Order Optical Nonlinearities in Open-Shell Chromophores. Planar Metal Complexes and Organic Radical Ion Aggregates", JOURNAL OF THE AMERICAN CHEMICAL SOCIETY, vol. 118, no. 50, 1 January 1996 (1996-01-01), pages 12747-12751, XP055024332, ISSN: 0002-7863, DOI: 10.1021/ja960561j
- I RATERA ET AL: "Nonlinear optical properties of open-shell polychlorotriphenylmethyl radicals", POLYHEDRON, vol. 22, no. 14-17, 1 July 2003 (2003-07-01), pages 1851-1856, XP055024330, ISSN: 0277-5387, DOI: 10.1016/S0277-5387(03)00138-4

## Description

### CROSS-REFERENCE TO RELATED APPLICATIONS

This application is a utility application, which claims benefit of U.S. Provisional Patent Application No. 61/418,136, filed November 30, 2010.

### BACKGROUND OF THE INVENTION

Polymeric electro-optic (EO) materials have demonstrated enormous potential for core application in a broad range of systems and devices, including phased array radar, satellite and fiber telecommunications, cable television (CATV), optical gyroscopes for application in aerial and missile guidance, electronic counter measure (ECM) systems, backplane interconnects for high-speed computation, ultrafast analog-to-digital conversion, land mine detection, radio frequency photonics, spatial light modulation and all-optical (light-switching-light) signal processing.

Nonlinear optic ("NLO") materials are capable of varying their first-, second-, third- and higher-order polarizabilities in the presence of an externally applied electric field or incident light (two-photon absorption). In telecommunication applications, the second-order polarizability (hyperpolarizability or β) and third-order polarizability (second-order hyperpolarizability or γ) are currently of great interest. The hyperpolarizability is related to the change of a NLO material's refractive index in response to application of an electric field. The second-order hyperpolarizability is related to the change of refractive index in response to photonic absorbance and thus is relevant to all-optical signal processing. The third-order polarizability relates to the change of refractive index in response to an intense light field. A more complete discussion of nonlinear optical materials may be found in D. S. Chemla and J. Zyss, Nonlinear optical properties of organic molecules and crystals, Academic Press, 1987 and
K.-S. Lee, Polymers for Photonics Applications I, Springer 2002, the entire contents of which are hereby incorporated by reference.

Many NLO molecules (chromophores) have been synthesized that exhibit high molecular electro-optic properties. The product of the molecular dipole moment (µ) and hyperpolarizability (β) is often used as a measure of molecular electro-optic performance due to the dipole's involvement in material processing. One chromophore originally evaluated for its extraordinary NLO properties by Bell Labs the 1960s, Disperse Red (DR), exhibits an electro-optic coefficient µβ ∼ 580x 10⁻⁴⁸ esu. Current molecular designs, including FTC, CLD and GLD, exhibit µβ values in excess of 10,000x10⁻⁴⁸ esu. See Dalton et al., "New Class of High Hyperpolarizability Organic Chromophores and Process for Synthesizing the Same", WO 00/09613, the entire contents of which are hereby incorporated by reference.

Nevertheless extreme difficulties have been encountered translating microscopic molecular hyperpolarizabilities (β) into macroscopic material hyperpolarizabilities (χ⁽²⁾). Molecular subcomponents (chromophores) must be integrated into NLO materials that exhibit: (i) a high degree of macroscopic nonlinearity; and, (ii) sufficient temporal, thermal, chemical and photochemical stability. Simultaneous solution of these dual issues is regarded as the final impediment in the broad commercialization of EO polymers in numerous government and commercial devices and systems.

The production of high material hyperpolarizabilities (χ⁽²⁾) is limited by the poor social character of NLO chromophores. Commercially viable materials must incorporate chromophores with the requisite molecular moment statistically oriented along a single material axis. In order to achieve such an organization, the charge transfer (dipolar) character of NLO chromophores is commonly exploited through the application of an external electric field during material processing which creates a localized lower-energy condition favoring noncentrosymmetric order. Unfortunately, at even moderate chromophore densities, molecules form multimolecular dipolarly-bound (centrosymmetric) aggregates that cannot be dismantled via realistic field energies. As a result, NLO material performance tends to decrease dramatically after approximately 20-30% weight loading. One possible solution to this situation is the production of higher performance chromophores that can produce the desired hyperpolar character at significantly lower molar concentrations.

Attempts at fabricating higher performance NLO chromophores have largely failed due to the nature of the molecular architecture employed throughout the scientific community. Currently all high-performance chromophores (e.g., CLD, FTC, GLD, etc.) incorporate protracted "naked" chains of alternating single-double.pi.-conjugated covalent bonds. Researchers such as Dr. Seth Marder have provided profound and detailed studies regarding the quantum mechanical function of such "bond-alternating" systems which have been invaluable to our current understanding of the origins of the NLO phenomenon and have in turn guided present-day chemical engineering efforts. Although increasing the length of these chains generally improves NLO character, once these chains exceed ~2 nm, little or no improvement in material performance has been recorded. Presumably this is largely due to: (i) bending and rotation of the conjugated atomic chains which disrupts the π-conduction of the system and thus reduces the resultant NLO character; and, (ii) the inability of such large molecular systems to orient within the material matrix during poling processes due to environmental steric inhibition. Improved chromophore architectures should exhibit at least two important characteristics: (i) a high degree of rigidity, and (ii) smaller conjugative systems that concentrate NLO activity within more compact molecular dimensions.

Long-term thermal, chemical and photochemical stability is the single most important issue in the construction of effective NLO materials. Material instability is in large part the result of three factors: (i) the increased susceptibility to nucleophilic attack of NLO chromophores due to molecular and/or intramolecular (CT) charge transfer or (quasi)-polarization, either due to high-field poling processes or photonic absorption at molecular and intramolecular resonant energies; (ii) molecular motion due
to photo-induced cis-trans isomerization which aids in the reorientation of molecules into performance-detrimental centrosymmetric configurations over time; and (iii) the extreme difficulty in incorporating NLO chromophores into a holistic cross-linked polymer matrix due to inherent reactivity of naked alternating-bond chromophore architectures. Improved chromophore architectures should: (i) exhibit improved CT and/or quasi-polar state stability; (ii) not incorporate structures that undergo photo-induced cis-trans isomerization; and (iii) be highly resistant to polymerization processes through the possible full-exclusion of naked alternating bonds. <page 4a>

### BRIEF SUMMARY OF THE INVENTION

The present invention relates, in general, to nonlinear optic chromophores comprising stabilized radical structures. Further are described methods for their production, nonlinear optical materials containing such chromophores, and the use of such materials in electro-optic devices. Different embodiments of the present invention relate to nonlinear optic chromophores comprising a stabilized radical of the formula (I'), (Ia) or (Ib) as shown in claims 1 to 3.

Described are further nonlinear optic, stabilized radical, chromophores of the general formula (I), and chromophore compositions which comprise a stabilized radical chromophore of the general formula (I): 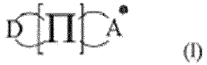 wherein D represents an organic electron donating group; A represents an organic electron accepting group having an electron affinity greater than the electron affinity of D; and Π represents a fused, offset, polycyclic, optionally heteroatom-containing, *pi-*conjugated core; wherein A is bound to the core at two atomic positions on the core such that at least a portion of A forms a ring fused to the core, wherein D is bound to the core at two atomic positions on the core other than the two atomic positions at which A is bound to the core such that at least a portion of D forms a ring fused to the core; and wherein the stabilized radical is optionally substituted with one or more pendant spacer groups.

Third-order Nanotechnologies, Inc. in "Third-Generation Electro-Optic Plastics", January 9, 2007, pages 1 to 12 provides an overview of the first- to fourth-generation of electro-optic plastics and presents an outlook beyond next generation.

Described are further nonlinear optic chromophore compositions which comprise mixtures of two or more stabilized radical chromophores of the general formula (I). These compositions include nonlinear optic chromophore compositions which comprise mixtures of nitro radicals of the general formula (I) and nitroxyl radicals of the general formula (I). The compositions also include nonlinear optic chromophore compositions which comprise mixtures of nitro radicals of the general formula (I) and/or nitroxyl radicals of the general formula (I), in addition to the "iso" version of such radicals, as described further below in this application. The compositions further include nonlinear optic chromophore compositions which comprise mixtures of nitro radicals of the general formula (I) and/or nitroxyl radicals of the general formula (I), and/or their "iso" versions, in addition to neutral (non-radicalized) chromophores referred to herein as Perkinamine™ chromophores.

Another embodiment of the present invention includes nonlinear optic chromophore compositions which comprise a mixture of two or more radicals selected from radicals of the general formula (I'), nitro radicals of the general formula (Ia) and a nitroxyl radicals of the general formula (Ib): 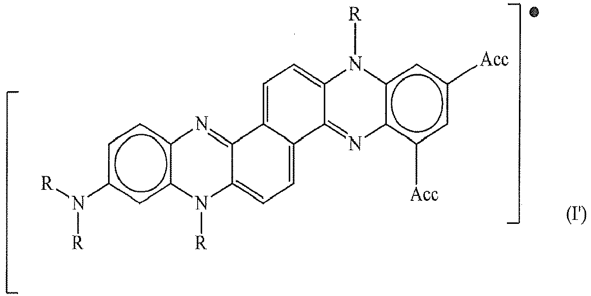 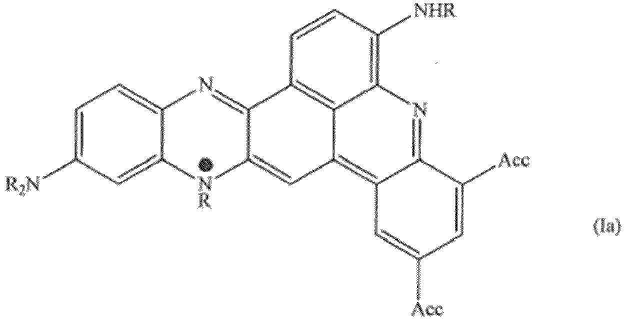 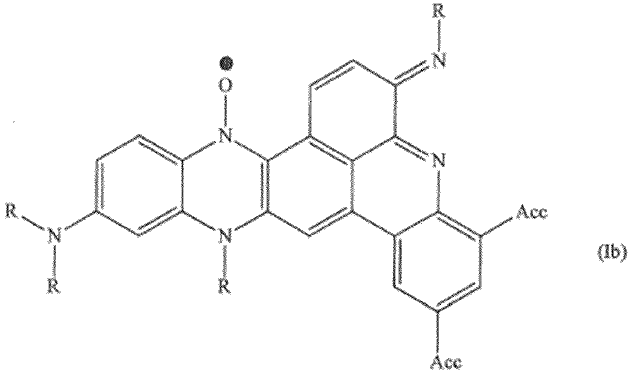 wherein each R and each Acc are independently as defined herein.

Nonlinear optic chromophores and compositions containing one or more such chromophores according to the present invention (referred to herein collectively as "nonlinear optic chromophores according to the present invention") surprisingly provide a significant improvement over existing chromophore architectures by exhibiting significantly greater electro-optic properties and also possessing a high degree of rigidity, and smaller conjugative systems that concentrate NLO activity within more compact molecular dimensions. Moreover, nonlinear optic chromophores according to the present invention exhibit improved CT and/or quasi-polar state stability; do not incorporate structures that undergo photo-induced cis-trans isomerization; and are highly resistant to polymerization processes through the possible full-exclusion of naked alternating bonds.

The nonlinear optic chromophores according to the present invention do not incorporate naked bond-alternating chains that are susceptible to bending or rotation. The central anti-aromatic conductors "pull" the molecule into a quasi-CT state; since aromaticity and non-CT states are both favorably low-energy conditions, charge transfer and aromaticity within the molecular systems described herein are set against each other within a competitive theater. This competitive situation is known as CAPP engineering or Charge-Aromaticity Push-Pull. As a result, the incorporation of anti-aromatic systems dramatically improves the conductive properties of the central n-conjugated bridge providing for smaller molecular lengths with significantly greater NLO property. Because all the systems described herein are aromatic in their CT state and quasi-aromatic in their intermediate quasi-polarized states, this structure can dramatically improve polar-state stability. Electronic acceptor systems are described herein which can also significantly improve excited-state and quasi-CT delocalization, making the overall systems less susceptible to nucleophilic attack. The heterocyclical nature of the systems described herein forbids the existence of photo-induced cis-trans isomerization which is suspected as a cause of both material and molecular degeneration. The nonlinear optic chromophores according to the present invention are devoid of naked alternating bonds that are reactive to polymerization conditions. Finally, the stabilized radical structure of the nonlinear optic chromophores according to the present invention provides significantly greater electro-optic properties than prior art chromophores.

Described are further methods of preparing nonlinear optic chromophores which comprise a stabilized radical of the general formula (I), the methods comprising: (i) providing a fused, polycyclic, optionally heteroatom-containing, *pi*-conjugated core having a first terminus and a second terminus, wherein the first terminus comprises a first terminus moiety which is reactive with an
electron donating group and wherein the second terminus comprises a second terminus moiety which is reactive with an electron accepting group; (ii) reacting the core with an electron donating group D such that the first terminus moiety and the electron donating group D undergo ring closure such that the electron donating group D is fused to the core at two atomic positions on the core to form a core-D intermediate; and (iii) subsequently reacting the core-D intermediate with an electron accepting group A, in the presence of a base, such that the second terminus moiety and the electron accepting group A undergo ring closure such that the electron donating group A is fused to the core at two atomic positions on the core, to form the stabilized radical of the general formula (I).

Described are further nonlinear optical materials which comprise stabilized radicals of the general formula (I) or mixtures incorporated within a matrix material. Suitable matrix materials can include various polymers, solutions, glass and others. Suitable methods for incorporating a stabilized radical of the general formula (I) or mixtures thereof into a polymer matrix material include: combining the chromophore radical with the polymer; electric field poling of the chromophore radical/polymer mixture to acentrically align chromophores; followed by crosslinking, curing, and/or hardening the chromophore radical-containing polymer. The chromophore radical can be physically incorporated into a polymer to provide a composite. The chromophore radical can be covalently incorporated into the polymer by, for example, attachment as a side chain or crosslinking. The chromophore radical can be crosslinked to the polymer in more than one position, for example, a double-ended crosslinked chromophore.

Described are further electro-optic devices which comprise a nonlinear optical material in accordance with the foregoing embodiments.

### BRIEF DESCRIPTION OF THE SEVERAL VIEWS OF THE DRAWING

The foregoing summary, as well as the following detailed description of the invention, may be better understood when read in conjunction with the appended drawings. For the purpose of assisting in the explanation of the invention, there are shown in the drawings representative embodiments which are considered illustrative. It should be understood, however, that the invention is not limited in any manner to the precise arrangements and instrumentalities shown.

In the drawings:
Fig. 1 is a depiction of a phase modulator in accordance with an embodiment of the present invention;
Fig. 2 is a depiction of a testing procedure for evaluation of a phase modulator as shown in Fig. 1; and
Fig. 3 is an image of a photomask architecture layout for a wafer used in a phase modulator as shown in Fig. 1.
Fig. 4 is a depiction of a degenerate four wave mixing (DFWM) testing procedure for evaluation of third-order NLO properties of thin film composites.

### DETAILED DESCRIPTION OF THE INVENTION

As used herein, the singular terms "a" and "the" are synonymous and used interchangeably with "one or more" and "at least one," unless the language and/or context clearly indicates otherwise. Accordingly, for example, reference to "a pendant spacer group" herein or in the appended claims can refer to a single pendant spacer group or more than one pendant spacer group. Additionally, all numerical values, unless otherwise specifically noted, are understood to be modified by the word "about."

Various core component structures, bridging groups and spacer moieties suitable for use in various embodiments of the present invention are described in published U.S. Patent Applications: US 2007/0260062, published on November 8, 2007; US 2007/0260063, published on November 8, 2007; US 2008/0009620, published on January 10, 2008; US 2008/0139812, published on January 12, 2008; and US 2009/0005561, published on January 1, 2009 (collectively referred to as "the prior publications"); the entire contents of each of which are hereby incorporated by reference.

In US 2008/0139812, the present inventors described a class of heterocyclic donor-acceptor chromophores referred to collectively by the present inventors as "Perkinamine™ chromophores." In exemplary synthesis schemes set forth in US 2008/0139812 (Paragraphs [0063] and [0064]), one of the final steps is a ring-closure reaction using picryl chloride and a geminal aromatic diamine. The present inventors have now surprisingly found that such processes can be at least partially controlled to yield ultimately either neutral perkinamines as in the prior publications, or a semi-reduced form that differs from the neutral perkinamines by the addition of one electron and one proton, that is, a protonated radical anion. In various preferred embodiments of the present invention wherein the electron accepting group comprises one or more nitro groups, the present application refers to the novel class of chromophores as "Perkinamine™ NR(s)", for "nitro radical(s)." In addition, while the "nitro radical" species are persistent radicals, stable under air-free conditions, we have found that a core amine functionality of these species is selectively oxidized to nitroxyl radicals which are stable under ambient conditions.

In contrast to the syntheses disclosed in the prior publications, the present inventors have found it advantageous to reverse the order of the penultimate and ultimate reactions, that is to form the electron accepting group ring last. When this is done, more control can be exerted on the final reaction to yield primarily perkinamine or primarily perkinamine NR depending on the nature of the base used to scavenge acid released during the ring closure.

In accordance with various embodiments of the present invention, virtually any base can be employed in the electron accepting group ring closure reaction, but preferably the base is non-nucleophilic. It is important that the electron accepting group ring closure reaction is carried out under dry conditions. Thus, solvents employed, such as, for example, ethanol, should be dry. Preferably, the reaction is carried out under
completely anhydrous conditions. It is preferred that the base is present during the electron accepting group ring closure reaction in stoichiometric excess. In addition, it is preferred that the electron accepting group ring closure reaction is carried out under an inert atmosphere.

It is preferred that the base employed in the electron accepting group ring closure reaction can include non-nucleophilic bases, such as, for example, potassium fluoride, sodium acetate and sodium carbonate. Preferred bases include potassium fluoride, sodium acetate and sodium carbonate. The most preferred base is sodium carbonate.

The electron accepting group ring closure reaction can be carried out above room temperature for any suitable period of time. It is preferred that the reaction may be carried out at 30 to 45 °C for a period of 3 to 4 hours.

Without being bound by theory, it is believed that perkinamines and perkinamine NRs are related as shown in the following diagram:

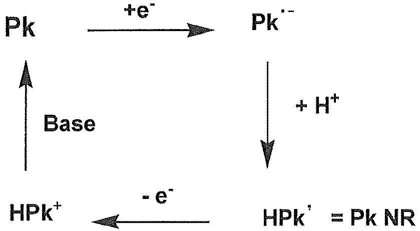

In the diagram, Pk stands for a neutral perkinamine, substituted with variable R spacer groups as described in the prior publications; and HPk^{•} is the protonated radical anion perkinamine NR.

Perkinamines as described in the prior publications, in general, have good nonlinear optical properties, especially the electro-optic property r₃₃ (the Pockels coefficient) which is critical for effective use of organic materials as electro-optic modulators for telecommunications. For example, Perkinamine 1 (shown below), as described in US 2008/0139812 at Paragraph [0063], where each R (*i.e*., "Sp") is a mesityl group, has an r₃₃ value which is about two times greater than that of a benchmark material synthesized by chemists at the University of Washington (Seattle) referred to as "CLD-1." The inventive stabilized radical chromophore analogous to Perkinamine 1 has an r₃₃ value which is 700% that of perkinamine 1. Thus, the inventive stabilized radical chromophores are more polarizable than neutral perkinamines.

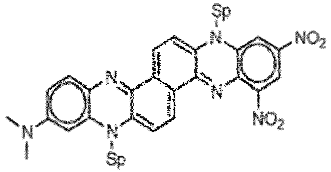

The inventive stabilized radical chromophores can be characterized as chemically different from the neutral perkinamines of the prior publications in a variety of ways including mass spectra, electronic spectra, nuclear magnetic resonance spectra, electron spin resonance spectroscopy, and electrochemical behavior.

Mass spectra of the inventive stabilized radical chromophores will exhibit a mass exactly one unit higher than a corresponding neutral perkinamine.

Electronic spectra will show similar strong charge transfer transitions for both neutral perkinamines and the inventive stabilized radical chromophores, but the transition for the inventive stabilized radical chromophores will be at a lower energy than the transition for the corresponding neutral perkinamine.

The neutral perkinamines of the prior publications are diamagnetic and will exhibit conventional and assignable NMR spectra. The inventive stabilized radical chromophores of the present invention, as radicals, are paramagnetic. Thus, the inventive stabilized radical chromophores will not provide conventional and assignable NMR spectra, but rather only broad signals.

Conversely, because the neutral perkinamines of the prior publications are diamagnetic, they do not exhibit an electron spin resonance spectrum. On the other hand, the inventive stabilized radical chromophores exhibit a strong ESR signal, characteristic of a free electron in a paramagnetic organic solid.

The inventive stabilized radical chromophores can be facilely oxidized in a one-electron oxidation which is entirely reversible on the time-scale of cyclic voltammetry. On the other hand, while the neutral perkinamines of the prior publications can be reduced, the process is not reversible. These results are consistent with the redox cycle shown above for Pk and Pk NR material families.

Persistent (or stable) free radicals are known, but they are rare. See "Persistent carbon-centered radicals," D. Griller and K. U. Ingold, Acc. Chem. Res., 1976, 9(1), pp. 13-19. Semireduced intermediates are known in the phenazine and phenothiazine dye families, but their lifetimes are in the sub-second range. See for example "Electron-transfer processes of dyes at semiconductor surfaces, X. Guangshi and C. Ciping in Encyclopedia of Surface and Colloid Science, Vol 3, p. 2311, edited by P. Sonansundaran, Taylor and Francis, London, 2006, the entire contents of which are hereby incorporated by reference. Nitroxyl radicals are well-described in a review by Rozantsev and Sholle (Russian Chem. Rev., 40 (3), 233 (1971).

As used herein, the term "halo," unless otherwise indicated, includes fluoro, chloro, bromo or iodo. Preferred halo groups are fluoro, chloro and bromo.

The term "alkyl," as used herein, unless otherwise indicated, includes saturated monovalent hydrocarbon radicals which may be straight, cyclic and/or branched. It is understood that for cyclic moieties at least three carbon atoms are required in said alkyl group.

The term "alkenyl," as used herein, unless otherwise indicated, includes monovalent hydrocarbon radicals having at least one carbon-carbon double bond and which may be straight, cyclic and/or branched.

The term "alkynyl," as used herein, unless otherwise indicated, includes monovalent hydrocarbon radicals having at least one carbon-carbon triple bond and which may be straight, cyclic and/or branched.

The term "alkoxy," as used herein, unless otherwise indicated, includes O-alkyl groups wherein "alkyl" is as defined above.

The term "aryl," as used herein, unless otherwise indicated, includes organic radicals derived from aromatic hydrocarbons by removal of one hydrogen, such as phenyl or naphthyl.

The term "heteroaryl," as used herein, unless otherwise indicated, includes organic radicals derived by removal of one hydrogen atom from a carbon atom in the ring of a heteroaromatic hydrocarbon, containing one or more heteroatoms independently selected from O, S, and N. Heteroaryl groups must have at least 5 atoms in their ring system and are optionally substituted independently with 0-2 halogen, trifluoromethyl, C₁-C₆ alkoxy, C₁-C₆ alkyl, or nitro groups.

The term "4-10 membered heterocyclic," as used herein, unless otherwise indicated, includes aromatic and non-aromatic heterocyclic groups containing one or more heteroatoms each selected from O, S and N, wherein each heterocyclic group has from 4-10 atoms in its ring system. Non-aromatic heterocyclic groups include groups having only 4 atoms in their ring system, but aromatic heterocyclic groups must have at least 5 atoms in their ring system. An example of a 4 membered heterocyclic group is azetidinyl (derived from azetidine). An example of a 5 membered heterocyclic group is thiazolyl and an example of a 10 membered heterocyclic group is quinolinyl. Examples of non-aromatic heterocyclic groups are pyrrolidinyl, tetrahydrofuranyl, tetrahydrothienyl, tetrahydropyranyl, tetrahydrothiopyranyl, piperidino, morpholino, thiomorpholmo, thioxanyl, piperazinyl, azetidinyl, oxetanyl, thietanyl, homopiperidinyl,
oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, ³H-indolyl and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups, as derived from the compounds listed above, may be C-attached or N-attached where such is possible. For instance, a group derived from pyrrole may be pyrrol-1-yl (N-attached) or pyrrol-3-yl (C-attached).

The term "saturated cyclic group" as used herein, unless otherwise indicated, includes non-aromatic, fully saturated cyclic moieties wherein alkyl is as defined above.

The phrase "acceptable salt(s)", as used herein, unless otherwise indicated, includes salts of acidic or basic groups which may be present in the compounds of the invention. The compounds of the invention that are basic in nature are capable of forming a wide variety of salts with various inorganic and organic acids. The acids that may be used to prepare pharmaceutically acceptable acid addition salts of such basic compounds of the invention are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate,
benzenesulfonate, p-toluenesulfonate and pamoate [i.e., 1,1'-methylene-bis-(2-hydroxy-3-naphthoate)] salts.

Those compounds of the invention that are acidic in nature are capable of forming base salts with various pharmacologically acceptable cations. Examples of such salts include the alkali metal or alkaline earth metal salts and particularly the sodium and potassium salts.

The term "solvate," as used herein includes a compound of the invention or a salt thereof, that further includes a stoichiometric or non-stoichiometric amount of a solvent bound by non-covalent intermolecular forces.

The term "hydrate," as used herein refers to a compound of the invention or a salt thereof, that further includes a stoichiometric or non-stoichiometric amount of water bound by non-covalent intermolecular forces.

Certain compounds as described above may have asymmetric centers and therefore appear in different enantiomeric forms. Further described is the use of all optical isomers and stereoisomers of the compounds of the invention and mixtures thereof. The compounds of the invention may also appear as tautomers. Thus, described is also the use of all such tautomers and mixtures thereof.

The subject invention also includes isotopically-labeled compounds, and the commercially acceptable salts thereof, which are identical to those recited in the various formulae described herein but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, sulfur, fluorine and chlorine such as ²H, ³H, ¹³C, ¹⁴C, ¹⁵N, ¹⁸0, ¹⁷0, ³⁵S, ¹⁸F, and ³⁶Cl, respectively. Compounds of the present invention and commercially acceptable salts of said compounds which contain the aforementioned isotopes and/or other isotopes of other atoms are within the scope of this invention. Certain isotopically-labeled compounds of the present invention, for example those into which radioactive isotopes such as ³H and
C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain advantages resulting from greater stability. Isotopically labeled compounds of this invention can generally be prepared by carrying out the procedures disclosed in the Schemes and/or in the Examples and Preparations below, by substituting a readily available isotopically labeled reagent for a non-isotopically labeled reagent.

As used herein, the term "nonlinear optic chromophore" (NLOC) refers to molecules or portions of a molecule that create a nonlinear optic effect when irradiated with light. The chromophores are any molecular unit whose interaction with light gives rise to the nonlinear optical effect. The desired effect may occur at resonant or nonresonant wavelengths. The activity of a specific chromophore in a nonlinear optic material is stated as its hyper-polarizability, which is directly related to the molecular dipole moment of the chromophore. The inventive stabilized radical NLO chromophores of the present invention are useful structures for the production of NLO effects.

The first-order hyperpolarizability (β) is one of the most common and useful NLO properties. Higher-order hyperpolarizabilities are useful in other applications such as all-optical (light-switching-light) applications. To determine if a material, such as a compound or polymer, includes a nonlinear optic chromophore with first-order hyperpolar character, the following test may be performed. First, the material in the form of a thin film is placed in an electric field to align the dipoles. This may be performed by sandwiching a film of the material between electrodes, such as indium tin oxide (ITO) substrates, gold films, or silver films, for example.

To generate a poling electric field, an electric potential is then applied to the electrodes while the material is heated to near its glass transition (Tg) temperature. After a suitable period of time, the temperature is gradually lowered while maintaining the poling electric field. Alternatively, the material can be poled by corona poling
method, where an electrically charged needle at a suitable distance from the material film provides the poling electric field. In either instance, the dipoles in the material tend to align with the field.

The nonlinear optical property of the poled material is then tested as follows. Polarized light, often from a laser, is passed through the poled material, then through a polarizing filter, and to a light intensity detector. If the intensity of light received at the detector changes as the electric potential applied to the electrodes is varied, the material incorporates a nonlinear optic chromophore and has an electro-optically variable refractive index. A more detailed discussion of techniques to measure the electro-optic constants of a poled film that incorporates nonlinear optic chromophores may be found in Chia-Chi Teng, Measuring Electro-Optic Constants of a Poled Film, in Nonlinear Optics of Organic Molecules and Polymers, Chp. 7, 447-49 (Hari Singh Nalwa & Seizo Miyata eds., 1997), except that in the event of any inconsistent disclosure or definition from the present application, the disclosure or definition herein shall be deemed to prevail.

The relationship between the change in applied electric potential versus the change in the refractive index of the material may be represented as its EO coefficient r₃₃. This effect is commonly referred to as an electro-optic, or EO, effect. Devices that include materials that change their refractive index in response to changes in an applied electric potential are called electro-optical (EO) devices.

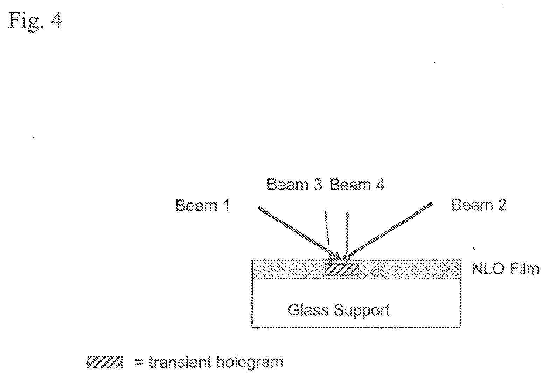

The second-order hyperpolarizability (γ) or third-order susceptibility (χ⁽³⁾), are the normal measures of third-order NLO activity. While there are several methods used to measure these properties, degenerate four-wave mixing (DFWM) is very common. See C. W. Thiel, "For- wave Mixing and Its Applications," http://www.physics.montana.edu.students.thiel.docs/FWMixing.pdf. Referring to Fig. 4 for example, a method of evaluating third-order NLO properties of thin films, known in the art as Degenerate Four Wave Mixing (DFWM), is depicted. In Fig. 4, Beams 1 and 2 are
picosecond, coherent pulses, absorbed by the NLO film deposited on a glass substrate. Beam 3 is a weaker, slightly delayed beam at the same wavelength as Beams 1 and 2. Beam 4 is the resulting product of the wave mixing, diffracted off of the transient holographic grating, produced by interferences of beams 1 and 2 in the NLO material of the film. Beam 3 can be a "control" beam at a telecom wavelength which produces a "signal" beam at a frequency not absorbed by the NLO material.

Described are further nonlinear optic chromophores which comprise a stabilized radical of the general formula (I): 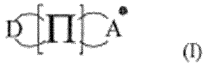 wherein D represents an organic electron donating group; A represents an organic electron accepting group having an electron affinity greater than the electron affinity of D; and Π represents a fused, offset, polycyclic, optionally heteroatom-containing, *pi-*conjugated core; wherein A is bound to the core at two atomic positions on the core such that at least a portion of A forms a ring fused to the core, wherein D is bound to the core at two atomic positions on the core other than the two atomic positions at which A is bound to the core such that at least a portion of D forms a ring fused to the core; and wherein the stabilized radical is optionally substituted with one or more pendant spacer groups.

In formula (I) A can represent any organic electron accepting group, so long as A is bound to the core at two atomic positions on the core such that at least a portion of A forms a ring fused to the core.

Examples of organic electron accepting groups suitable for incorporation into the chromophores of Formula (I) include, but are not limited to, the following structures, wherein the dashed lines represent the two atomic positions at which A forms a ring fused to the core: 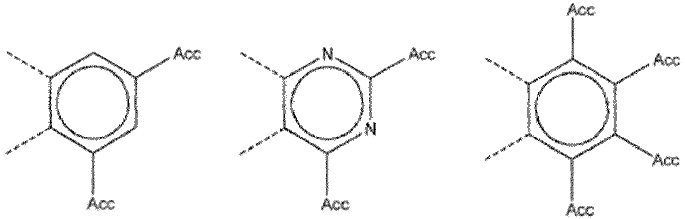 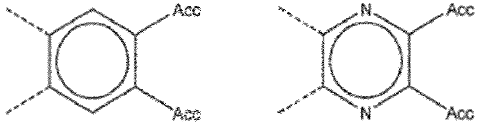 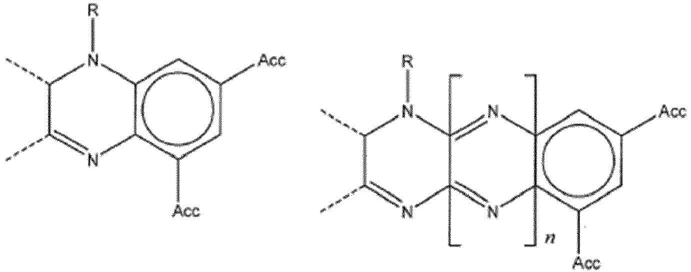 wherein each Acc independently represents an electron accepting moiety, R represents a pendant spacer group, and 0 < n < 5. Each Acc preferably independently represents an electron accepting moiety selected from CN, NO₂, and SO₂R. Most preferably, each Acc represents NO₂.

In formula (I) D can represent any organic electron donating group, so long as D is bound to the core at two atomic positions on the core other than the two atomic positions at which A is bound to the core such that at least a portion of D forms a ring fused to the core.

Examples of organic electron donating groups suitable for incorporation into the chromophores of Formula (I) include, but are not limited to, the following structures, wherein the dashed lines represent the two atomic positions at which D forms a ring fused to the core: 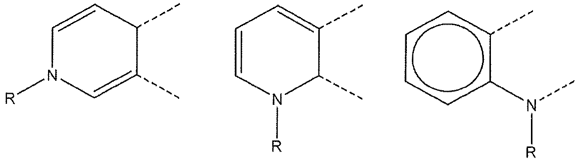 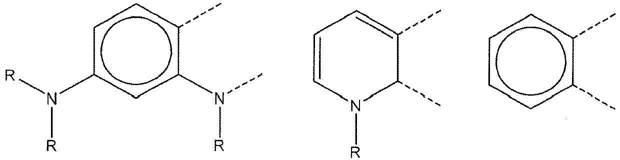 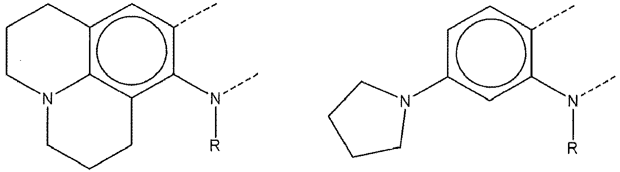 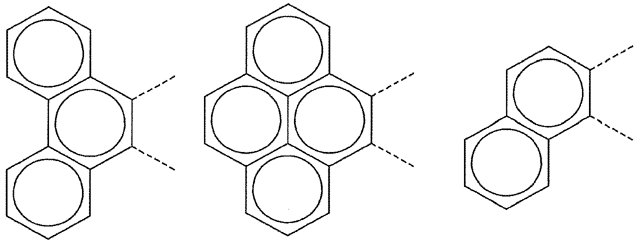 wherein each R independently represents a pendant spacer group.

Π represents a fused, offset, polycyclic, optionally heteroatom-containing, *pi*-conjugated core. Core structures are "*pi*-conjugated" meaning that the core structure contains at least two double bonds separated by a single bond, and preferably more than two double bonds each separated by a single bond. Core structures
are polycyclic and fused meaning that the core structure contains at least two rings which share two atoms between the two rings.

Specific core structures Π include those of the general formulae IIa, IIb, IIb' and IIc: 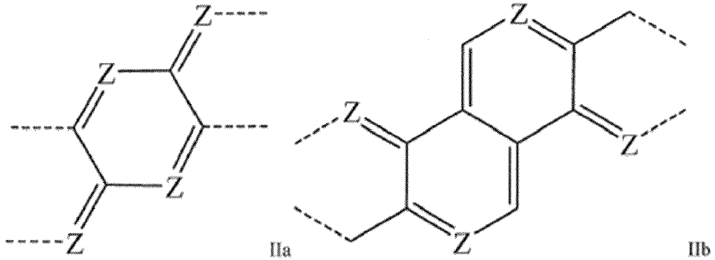 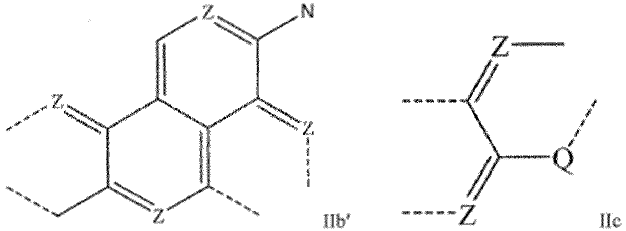 wherein each Z independently represents N, CH or CR; wherein R represents a pendant spacer group; and wherein each dashed line independently represents a chemical bond to another atom within the chromophore; and Q represents O, S, NH or NR.

Core structures are offset. As used herein, the term "offset" refers to a geometric (as opposed to optic) nonlinear character of the core structure. The term "offset" is used herein to refer to the geometric nonlinear character of the structures to avoid confusion with the optical nonlinear properties of the chromophores. Thus, herein, "offset" refers to a polycyclic, fused structure which contains a dispositional shift in the alignment of the
24 fused rings. For example, with reference to the preceding preferred core structures of formulae IIa, IIb, and IIc, the "offset" character can be explained and high-lighted with reference to the following structures containing jointed lines depicting the dispositional shift, or non-linear geometry thereof:

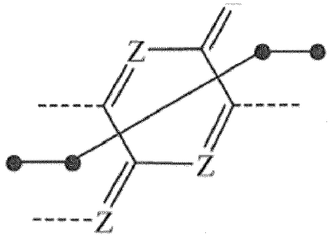

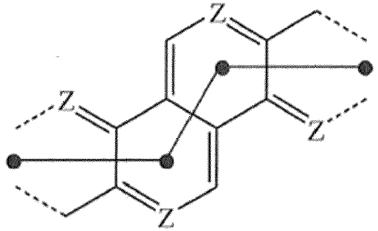

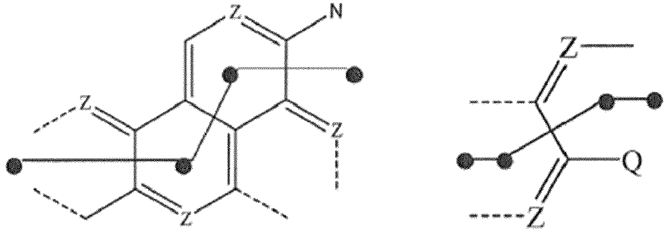

Additional core structures Π include those which contain more than one of the structures of formulae IIa, IIb, lIb' and/or IIc, which may either be bound directly to one another to form a linking ring or may be bound to one another by an additional optionally polycyclic, optionally heteroatom-containing ring structure.

Core structures can further comprise a first bridging group π¹ such that the two atomic positions at which D is bound are part of the first bridging group π¹. Core structures can further comprise a second bridging group π² such that the two atomic positions at which A is bound are part of the second bridging group π². Core structures can further comprise a first bridging group π¹ and a second bridging group π². Various suitable bridging groups are described m the prior publications.

Nonlinear optic chromophores according to the present invention can further comprise one or more pendant spacer groups bound to the core, first bridging group, second bridging group, electron donating group and/or electron accepting group. Pendant spacer groups in accordance with the present invention are generally nonreactive moieties which extend outward from the chromophore and create steric hindrance (i.e., "spacing") between two or more of the chromophore molecules in a material containing the chromophores, and thus serve to prevent aggregation during and after poling.

Suitable pendant spacer groups R can include spacer systems of the Formula IV: 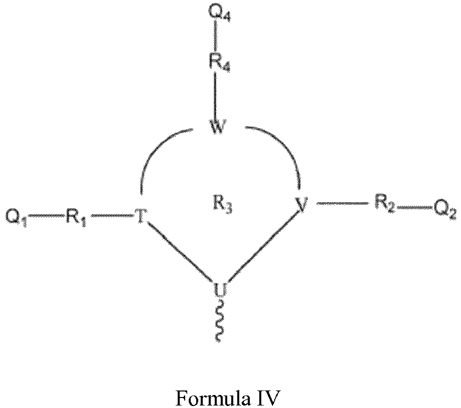 or an acceptable salt thereof; wherein R₃ is a C₆-C₁₀ aryl, C₆-C₁₀ heteroaryl, 4-10 membered heterocyclic or a C₆-C₁₀ saturated cyclic group; 1 or 2 carbon atoms in the foregoing cyclic moieties are optionally substituted by an oxo (=O) moiety; and the foregoing R3 groups are optionally substituted by 1 to 3 R₅ groups; R₁ and R₂ are independently selected from the list of substituents provided in the definition of R₃, a chemical bond ( - ), (CH₂)ₜ(C₆-C₁₀ aryl) or (CH₂)ₜ(4-10 membered heterocyclic), t is an integer ranging from 0 to 5, and the foregoing R₁ and R₂ groups are optionally substituted by 1 to 3 R₅ groups; R₄ is independently selected from the list of substituents provided in the definition of R₃, a chemical bond ( - ), or hydrogen; R₄ is independently selected from the list of substituents provided in the definition of R₃, a chemical bond ( - ), or hydrogen; each Q¹, Q², and Q⁴ is independently selected from hydrogen, halo, C₁- C₁₀alkyl, C₂-C₁₀ alkenyl, C₂-C₁₀ alkynyl, nitro, trifluoromethyl, trifluoromethoxy, azido, -OR⁵, -NR⁶C(O)OR⁵, -NR⁶SO₂R⁵, -SO₂NR⁵R⁶, -NR⁶C(O)R⁵,-C(O)NR⁵R⁶, -NR⁵R⁶, -S(O)ⱼR⁷ wherein j is an integer ranging from 0 to 2,-NR⁵(CR⁶R⁷)ₜOR⁶, -(CH₂)ₜ(C₆-C₁₀ aryl), -SO₂(CH₂)ₜ(C₆-C₁₀ aryl), -S(CH₂)ₜ(C₆-C₁₀ aryl), -O(CH₂)ₜ(C₆-C₁₀ aryl), -(CH₂)ₜ(4-10 membered heterocyclic), and -(CR⁶R⁷)ₘOR⁶, wherein m is an integer from 1 to 5 and t is an integer from 0 to 5; with the proviso that when R⁴ is hydrogen Q⁴ is not available; said alkyl group optionally contains 1 or 2 hetero moieties selected from O, S and -N(R⁶)- said aryl and heterocyclic Q groups are optionally fused to a C₆-C₁₀ aryl group, a C₅-C₈ saturated cyclic group, or a 4-10 membered heterocyclic group; 1 or 2 carbon atoms in the foregoing heterocyclic moieties are optionally substituted by an oxo (=0) moiety; and the alkyl, aryl and heterocyclic moieties of the foregoing Q groups are optionally substituted by 1 to 3 substituents independently selected from nitro, trifluoromethyl, trifluoromethoxy, azido, -NR⁶SO₂R⁵, -SO₂NR⁵R⁶, -NR⁶C(O)R⁵, -C(O)NR⁵R⁶, -NR⁵R⁶, -(CR⁶R⁷)ₘOR⁶ wherein m is an integer from 1 to 5, -OR⁵ and the substituents listed in the definition of R⁵; each R⁵ is independently selected from H, C₁-C₁₀ alkyl,-(CH₂)ₜ(C₆-C₁₀ aryl), and -(CH₂)ₜ(4-10 membered heterocyclic), wherein t is an integer from 0 to 5; said alkyl group optionally includes 1 or 2 hetero moieties selected from O, S and -N(R⁶)- said aryl and heterocyclic R⁵ groups are optionally fused to a C₆-C₁₀ aryl group, a C₅-C₈ saturated cyclic group, or a 4-10 membered heterocyclic group; and the foregoing R⁵ substituents, except H, are optionally substituted by 1 to 3 substituents independently selected from nitro, trifluoromethyl, trifluoromethoxy, azido, - NR⁶C(O)R⁷, -C(O)NR⁶R⁷, -NR⁶R⁷, hydroxy, C₁-C₆ alkyl, and C₁-C₆ alkoxy; each R⁶ and R⁷ is independently H or C₁-C₆ alkyl; T, U and V are each independently selected from C (carbon), O (oxygen), N (nitrogen), and S (sulfur), and are included within R³; T, U, and V are immediately adjacent to one another; and W is any non-hydrogen atom in R³ that is not T, U, or V.

Additional suitable pendant spacer groups R can include hydrogen, halo, C₁-C₁₀ alkyl, C₂-C₁₀ alkenyl, C₂-C₁₀ alkynyl, nitro, trifluoromethyl, trifluoromethoxy, azido, -OR⁵, -NR⁶C(O)OR⁵, -NR⁶S0₂R⁵, -S0₂NR⁵R⁶, -NR⁶C(O)R⁵,-C(0)NR⁵R⁶, -NR⁵R⁶, -S(0)ⱼR⁷ wherein j is an integer ranging from 0 to 2,-NR⁵(CR⁶R⁷)ₜOR⁶, -(CH₂)ₜ(C₆-C₁₀ aryl), -(CH₂)ₜSO₂(CH₂)ₜ(C₆-C₁₁ aryl), -S(CH₂)ₜ(C₆-C₁₀ aryl), -O(CH₂)ₜ(C₆-C₁₀ aryl), -(CH₂)ₜ(4-10 membered heterocyclic), and -(CR⁶R⁷)ₘ0R⁶, wherein m is an integer from 1 to 5 and t is an integer from 0 to 5; said alkyl group optionally contains 1 or 2 hetero moieties selected from O, S and -N(R⁶)-, wherein R5, R6 and R7 are as defined above, and wherein the aryl and heterocyclic moieties may optionally be further substituted with any of the aforementioned groups.

Particularly preferred pendant spacer groups R include mesityl groups, 2-ethylhexyl groups, wo-propyl groups, cyclohexyl groups, halogenated aromatics (e.g., dihalophenyls, and in particular, 3,5-dichlorophenyl), and -(CH₂)ₜSO2(CH₂)ₜ(C₆-C₁₀ aryl) groups, such as groups of the formula:

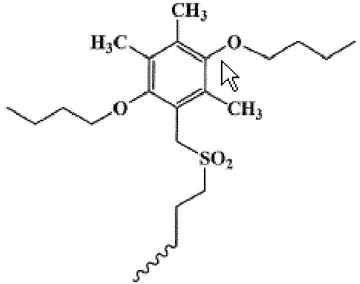

Further is described: Methods of preparing nonlinear optic chromophores which comprise a stabilized radical of the general formula (I) include:
(i) providing a fused, polycyclic, optionally heteroatom-containing, (*pi*-conjugated core having a first terminus and a second terminus, wherein the first terminus comprises a first terminus moiety which is reactive with an electron donating group and wherein the second terminus comprises a second terminus moiety which is reactive with an electron accepting group; (ii) reacting the core with an electron donating group D such that the first terminus moiety and the electron donating group D undergo ring closure such that the electron donating group D is fused to the core at two atomic positions on the core to form a core-D intermediate; and (iii) subsequently reacting the core-D intermediate with an electron accepting group A, in the presence of a base, such that the second terminus moiety and the electron accepting group A undergo ring closure such that the electron donating group A is fused to the core at two atomic positions on the core, to form the stabilized radical of the general formula (I)..

The nonlinear optic chromophore of the present invention can be used in nonlinear optical materials wherein the nonlinear optic chromophore comprises a stabilized radical
incorporated within a matrix material. Suitable matrix materials can include polymers, such as, for example: poly(methylmethacrylate)s (PMMA); polyimides; polyamic acid; polystyrenes; poly(urethane)s (PU); and amorphous polycarbonates (APC). Preferably, the matrix material comprises a poly(methylmethacrylate). Particularly preferred poly(methylmethacrylate)s have a molecular weight of about 120,000 and a glass transition temperature Tg of about 85-165°C.

The nonlinear optic chromophores of the present invention are generally incorporated within the matrix material at a loading of 1 % to 50 % by weight, based on the entire nonlinear optical material, more preferably at a loading of 2 % to 35 % by weight, and most preferably at a loading of 3 % to 35 % by weight. Nonlinear optical materials can be in the form of solid thin films, optionally disposed on a surface of another material. In general, nonlinear optical materials include all existing known forms of such materials, but wherein the optical chromophore incorporated within the matrix material comprises a stabilized radical.

The nonlinear optical material of the present invention can be used in electro-optic devices. Electro-optic devices and/or systems include phased array radar, satellite and fiber telecommunications, cable television (CATV), optical gyroscopes for application in aerial and missile guidance, electronic counter measure systems (ECM) systems, backplane interconnects for high-speed computation, ultrafast analog-to-digital conversion, land mine detection, radio frequency photonics, spatial light modulation and all-optical (light-switching-light) signal processing, wherein such devices include a nonlinear optical material according to the present invention. Moreover, the extremely broad absorption spectrum of nonlinear optic chromophores according to the present invention, which essentially covers the entire UV-visable-near infrared region from 250 nm to 1800 nm at high extinction coefficient, indicates that nonlinear optical
materials according to the present invention can also be used in solar conversion and photovoltaic devices.

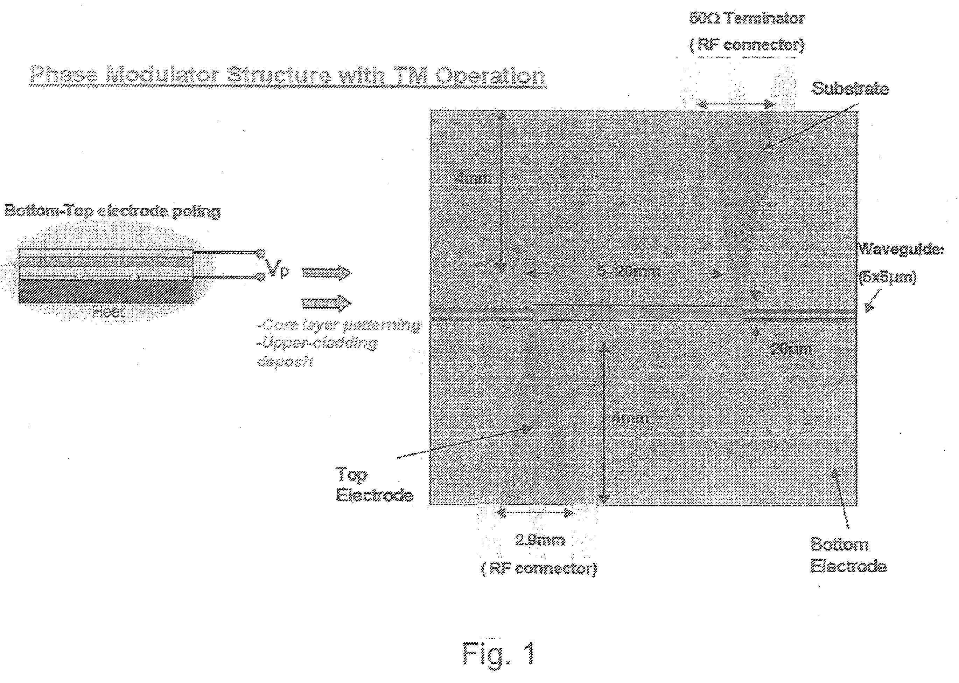

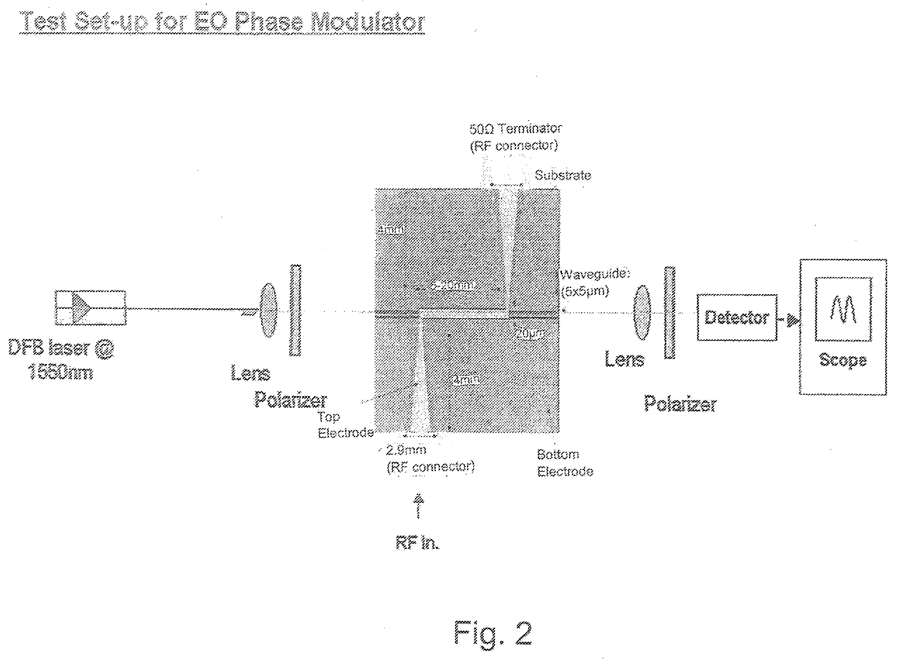

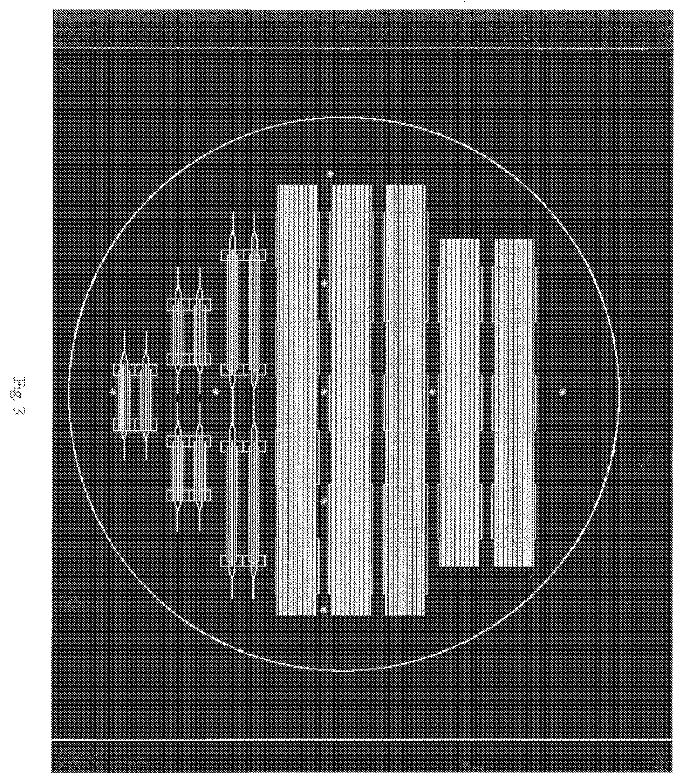

A specific electro-optic device includes electro-optic modulators for telecommunications, wherein the modulator comprises a nonlinear optical material according to the present invention. Referring to Fig. 1, a phase modulator design in accordance with one embodiment of the invention is shown. The waveguide in Fig. 1 comprises a nonlinear optical material according to the present invention, wherein the material comprises stabilized free radicals of Synthesis Example 1 (at 4.5% loading), incorporated in polymethylmethactylate ( MMA) having a molecular weight of about 120,000. Referring to Fig. 2, a set-up for evaluating the performance of such a phase modulator is depicted. Referring to Fig. 3, an image of a photomask architecture descriptive of the overall layout of a wafer used in such a device is depicted.

Another device, this one an all-optical one, is depicted in Figure 4. This device can be used for optical switching, parametric amplification and other all-optical applications of the third-order hyperpolarizability.

The invention will now be described in further detail with reference to the following non-limiting examples.

### EXAMPLES

### Synthesis Example 1:

A nonlinear optical chromophore according to an embodiment of the present invention was synthesized according to the following reaction scheme:

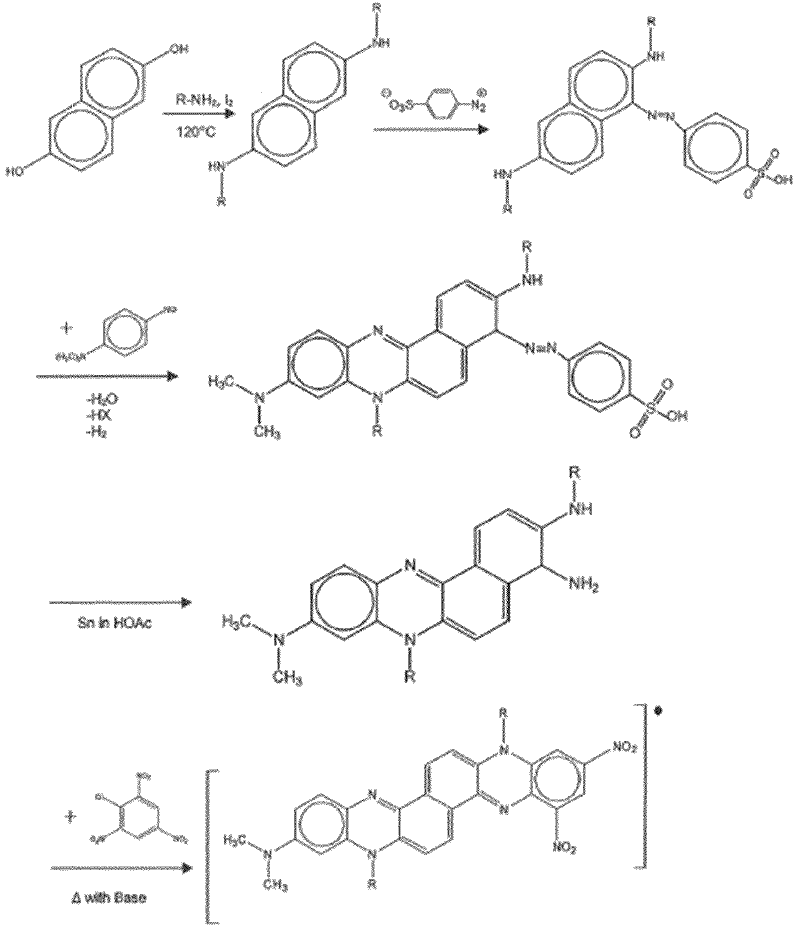

In the reaction scheme set forth above, each R represented a mesityl (*i.e*., 2,4,6-trimethylphenyl) group. Similar syntheses can be carried out according to this and other disclosed reaction schemes but with different R groups by modifying the first step to employ a different animated R group (*i.e*., R-NH₂). Thus, for example, in place of mesitylamine, the reaction scheme can be carried out with 2-ethylhexylamine so that each R group is a 2-ethylhexyl group. Also, for example, in place of mesitylamine, the reaction scheme can be carried out with cyclohexylamine so that each R group is a cyclohexyl group, etc. Additionally, as will be understood by those of ordinary skill in the art, while various structures and intermediates shown in this and other syntheses described herein contain static bond representations, the various intermediates and final stabilized radical structures exist in resonance forms with specific double bond locations that may vary.

In the synthesis example shown, the ring-closed product of the final step is thought to be produced in two stages. The first step constitutes a nucleophilic attack by the primary amine on the chloro group of the picryl chloride. This intermediate is then attacked by the remaining secondary, substituted, amino group to produce the product shown. We have found that an alternative path can be followed as well. After the intitial nucleophilic attack, the nitro group can react with a carbon atom on the aromatic ring below the site of the previous attack. This produces the structure shown below, referred to herein as an "iso"Perkinamine Nitro Radical:

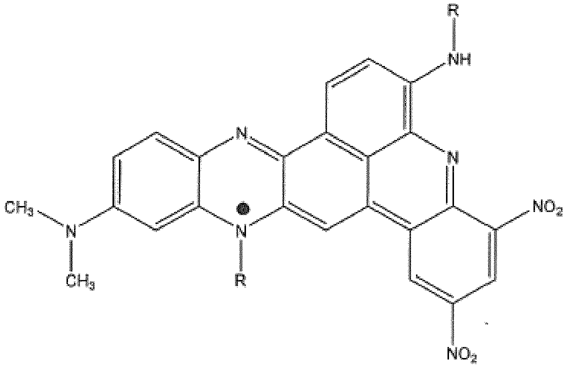

Also, oxidation of the Nitro Radical with oxygen (air) slowly produces the IsoPerkinamine Nitroxyl Radical:

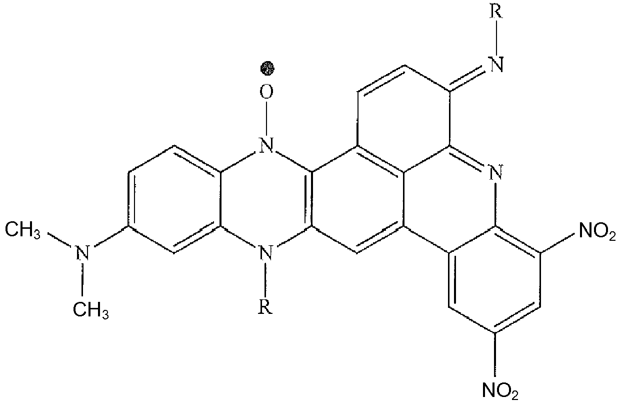

### Synthesis Example 2:

A second example of synthesis of a nitroxyl radical in the Perkinamine family is related below, in which each spacer (R) group is a cyclohexyl group. Bis-2,6-dicyclohexylaminonaphthalene is prepared by reaction of 2,6-dihydroxynaphthalene and cyclohexylamine, wherein R-NH₂ represents cyclohexylamine, and the remainder of the reactions of the general scheme shown in Synthesis Example 1 are carried out. The final step is described below.

0.200 g (0.397 mmol) of a compound of the following formula wherein each R represents a cyclohexyl group: 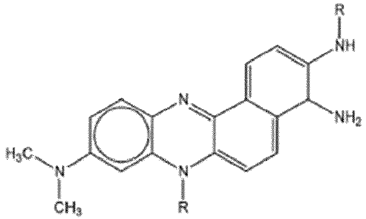 was added to 0.042 g (0.397 mmol) of anhydrous sodium carbonate in 9 mL of dry acetonitrile. This was left to stir for 5 min. before 0.180 g (0.727 mmol) of picryl chloride was added to the mixture. The reaction was heated to 85°C for 18 hours at which point, a black precipitate forms out of solution. Acetonitrile was removed in vacuo and the black solids were washed with 50 mL of water, then filtered. The mother liquor is a green-yellow color. The solids were washed with 20 mL of diethylether. The solid was collected and dried *in vacuo* to yield 0.207 g of material.

1H NMR (dDMSO): Consists of very broad signals at high concentration, with three sets of AB coupling groups. Green solution.

EPR (DMSO): strong doublet of triplets centered at 3365 G.

### Synthesis Example 3 (Reference):

A nonlinear optical chromophore is synthesized according to the following reaction scheme:

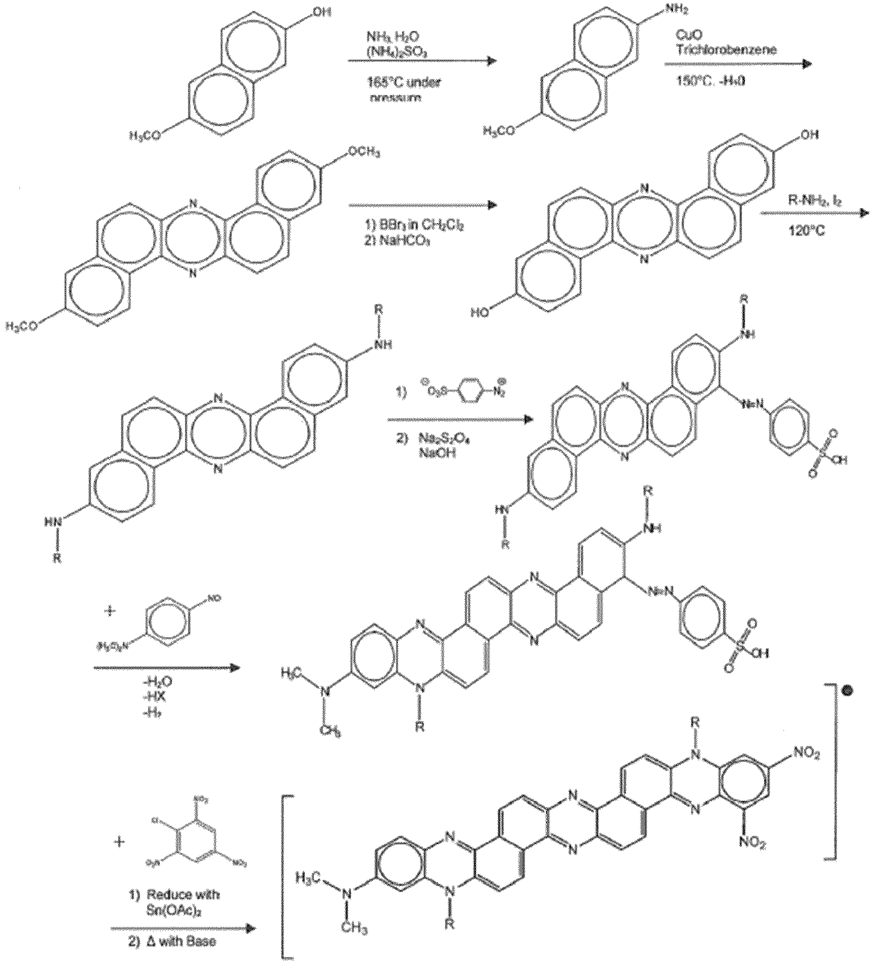

### Synthesis Example 4 (Reference):

A nonlinear optical chromophore is synthesized according to the following reaction scheme:

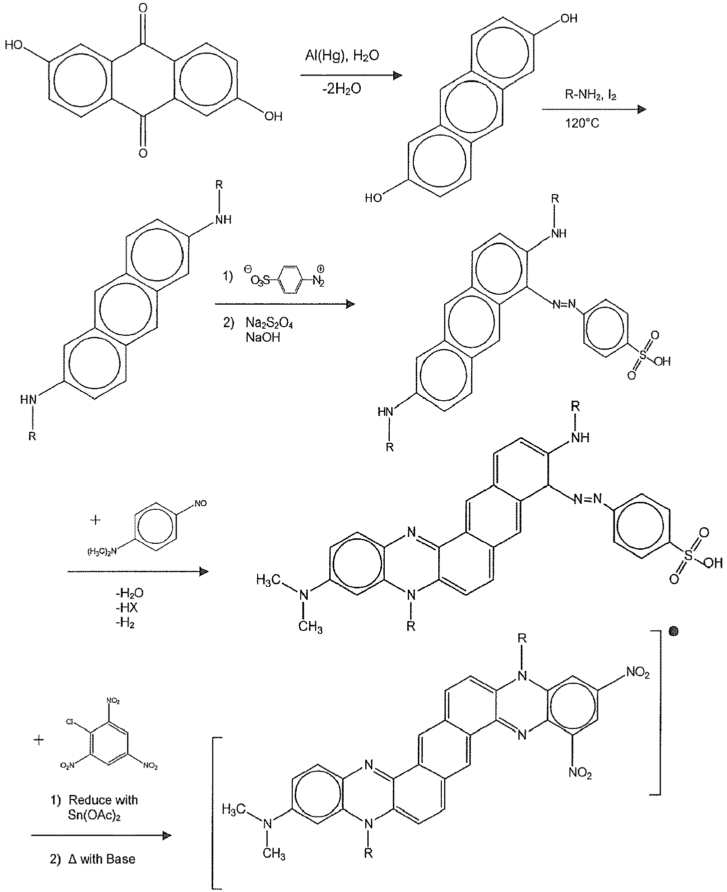

### Evaluation Example 1:

The nonlinear optical chromophore of Synthesis Example 1, (referred to in this Evaluation Example as "Perkinamine™ Nitro Radical" or "PNR") was evaluated via its mass spectrum, electronic spectrum, nuclear magnetic resonance spectrum, electron spin resonance spectroscopy, electrochemical behavior and DFWM response.

PNR exhibits a mass exactly one unit higher than the similar, but unradicalized, chromophore described in US 2008/0139812 at paragraph [0063] with R representing mesityl ("Perkinamine™ 1 "), thus indicating the presence of a protonated radical anion.

Electronic spectrum: Perkinamine™ 1 is a molecule with strong charge transfer transition at about 950 nm in acetonitrile. It is very solvatochromically shifted by changing solvent polarity. PNR is also solvatochromic, but its charge transfer band is in the mid-infrared region, at significantly lower energy than Perkinamine 1. PNR exhibits a strong charge transfer transition at about 1200 nm in acetonitrile.

Nuclear magnetic resonance spectrum: Perkinamine 1 has a conventional and assignable NMR spectrum. PNR does not provide a spectrum, only broad signals. The lack of a conventional and assignable NMR spectrum is presumably because PNR is paramagnetic which is evidence of its radical character.

Electron spin resonance ("ESR") spectroscopy: Perkinamine 1 does not show an ESR spectrum, presumably because it is diamagnetic. Solid PNR shows a strong ESR signal at g ~ 2.0, characteristic of a free electron in a paramagnetic organic solid. In solution (DMSO) the signal is a doublet of triplets characteristic of nitroxly radicals. Spin counting versus a standard indicated the sample was 20-40% radical in nature, with variability occurring batch to batch.

Electrochemical behavior: PNR can be facilely oxidized at +0.25 volts versus an Ag/Ag⁺ electrode. It is a one-electron oxidation and it is entirely reversible on
the time-scale of cyclic voltammetry. Perkinamine 1 can be reduced at about -0.1 v versus the Ag/Ag⁺ electrode, but the process is not reversible.

When the PNR of the present invention was tested for DFWM response, a thin film (3 microns in the polycarbonate binder APC containing 8.3 weight percent PNR) was exposed to counter-propagating coherent picosecond beams of near infrared laser light at circa 1150 nm. The transient holographic diffraction grating thus produced was exposed to a third beam (*see*, Figure 4) of lower power. The third beam diffracted and the power of the diffracted beam was measured. When the measured power of the diffractive beam was compared to the power diffracted from a 1 mm fused quartz standard, under identical conditions the power ratio was 4.6 to 7.9 in several experiments. The χ⁽³⁾ of fused silica is well known, and can be used to calculate the χ⁽³⁾ of the PNR sample to be 2600 times that of fused silica, or 4 x 10⁻¹⁹ m² V². Knowing the number density of PNR molecules in the thin film sample, the molecular value of γ can be determined to be 3000 x 10⁻⁴⁸ m⁵/V². This value is about 100 times larger than other small organic molecules and shows the efficacy of the PNR family for third-order NLO activity. The optical response was found to occur within the picosecond pulse width of the laser used for the experiment.

## Claims

1. A nonlinear optic chromophore comprising a stabilized radical of the general formula 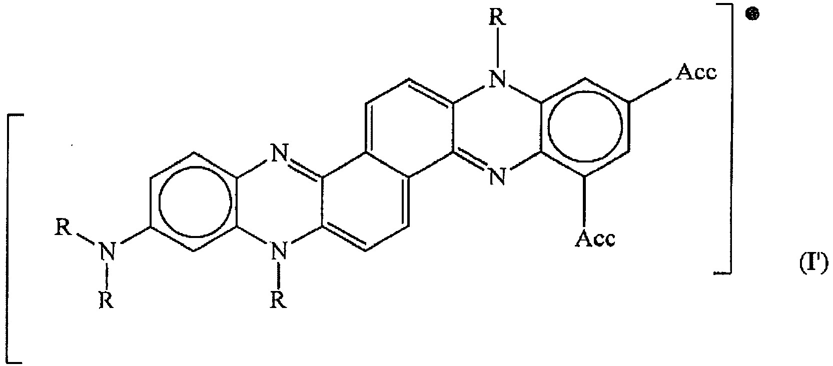 wherein each R independently represents a pendant spacer group and each Acc represents an electron accepting group.

2. A nonlinear optic chromophore comprising a stabilized radical of the general formula 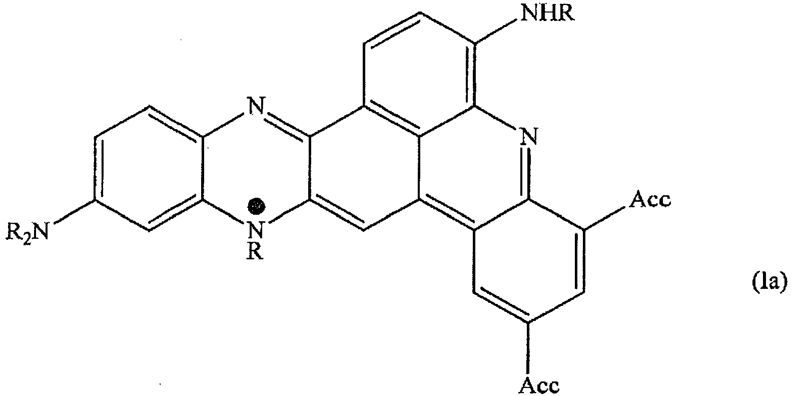 wherein each R independently represents a pendant spacer group and each Acc represents an electron accepting group.

3. A nonlinear optic chromophore comprising a stabilized radical of the general formula 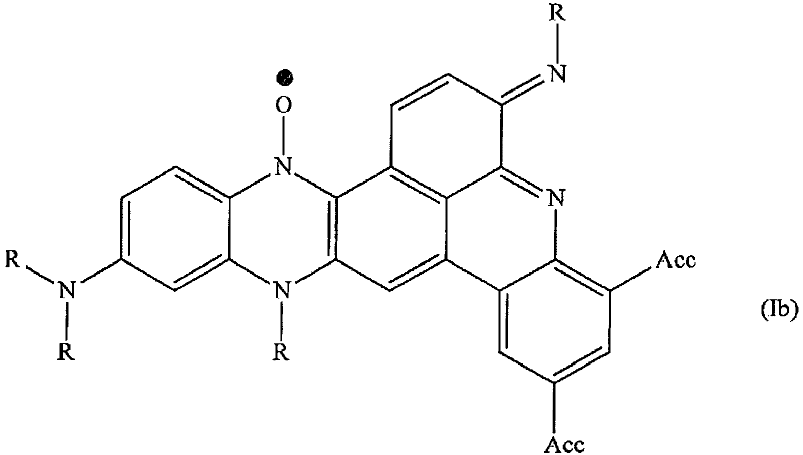 wherein each R independently represents a pendant spacer group and each Acc represents an electron accepting group.

4. The chromophore according to claim 1, wherein each R independently represents a moiety selected from the group consisting of mesityl,2-ethylhexyl and a structure of the general formula, wherein the structure is bound to the chromophore at the

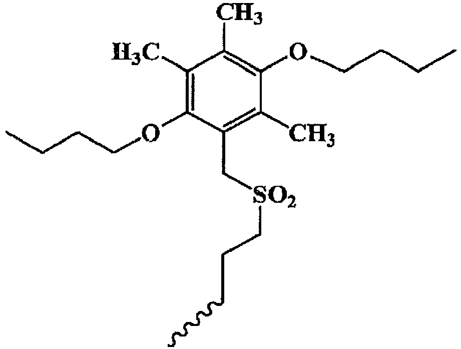

5. The chromophore according to claim 1, wherein each R represents a mesityl group.

6. The chromophore according to claim 1, wherein each R represents a 2-ethylhexyl group.

7. The chromophore according to claim 1, wherein each R represents a cyclohexyl group.

8. A nonlinear optical chromophore composition comprising a mixture of two or more stabilized radicals selected from the group consisting selected from radicals of the general formula (I'), nitro radicals of the general formula (Ia) and a nitroxyl radicals of the general formula (Ib): 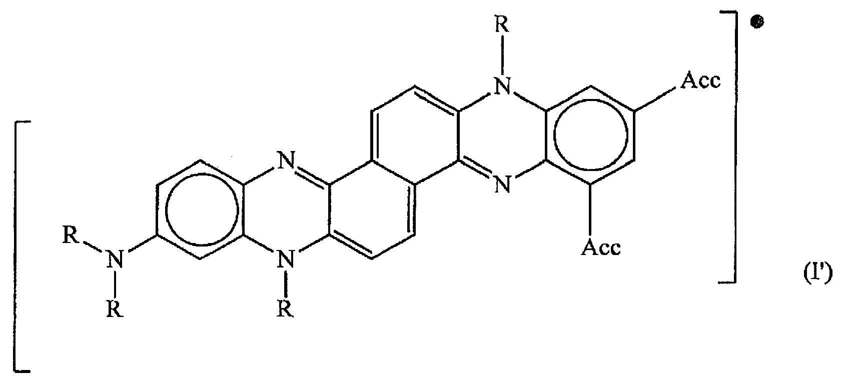 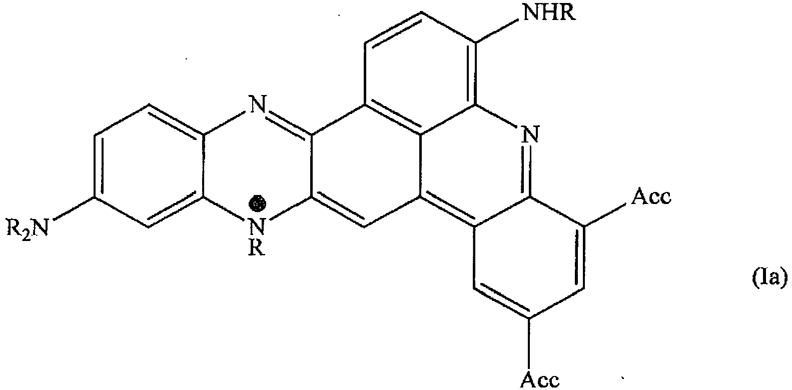 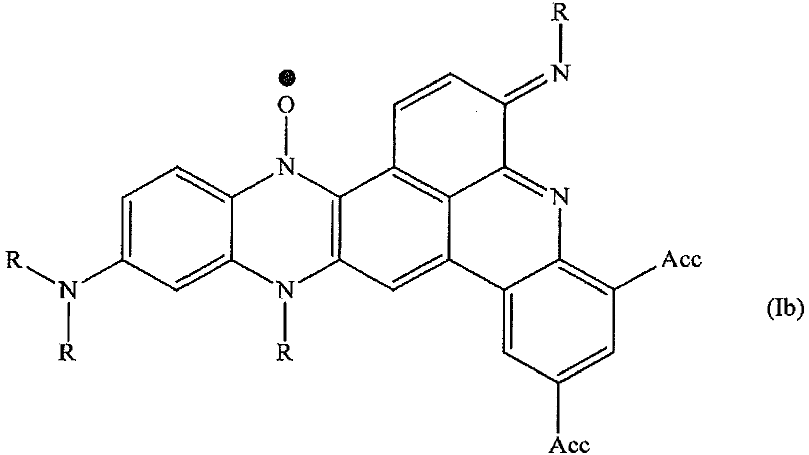 wherein each R independently represents a pendant spacer group and each Acc represents an electron accepting group.

## Patentansprüche

1. Nichtlinearer optischer Chromophor, umfassend ein stabilisiertes Radikal der allgemeinen Formel 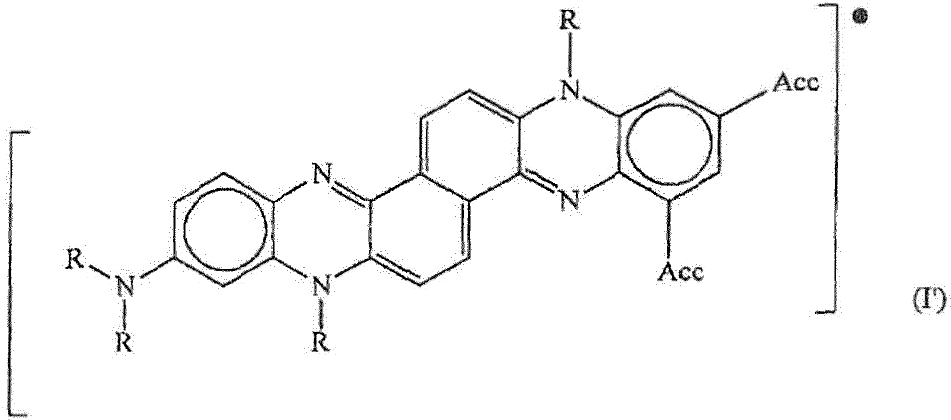 worin jedes R unabhängig eine Seitenabstandhaltergruppe und jedes Acc eine Elektronenakzeptorgruppe darstellt.

2. Nichtlinearer optischer Chromophor, umfassend ein stabilisiertes Radikal der allgemeinen Formel 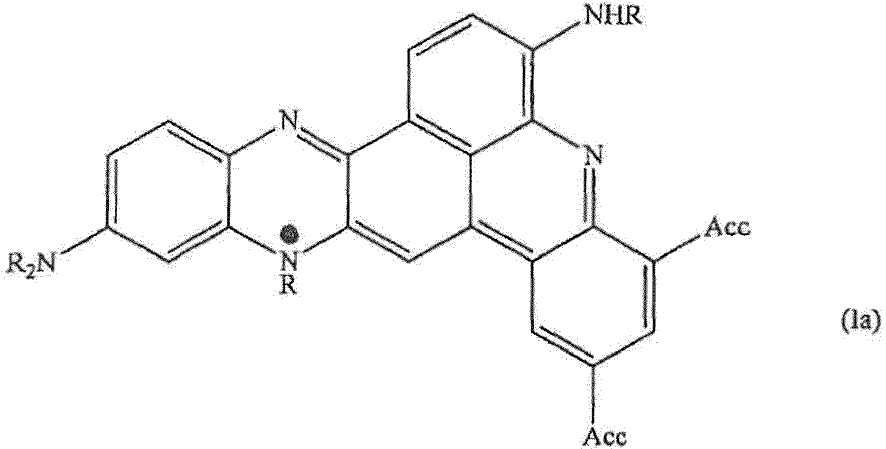 worin jedes R unabhängig eine Seitenabstandhaltergruppe und jedes Acc eine Elektronenakzeptorgruppe darstellt.

3. Nichtlinearer optischer Chromophor, umfassend ein stabilisiertes Radikal der allgemeinen Formel 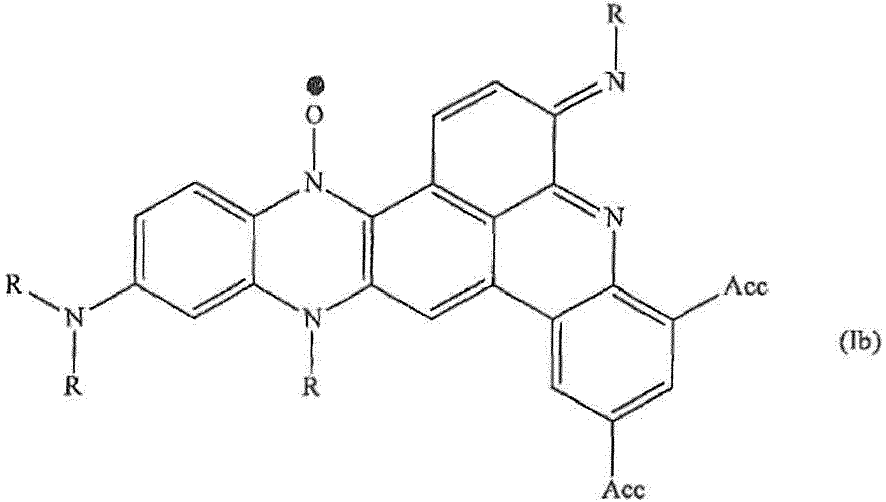 worin jedes R unabhängig eine Seitenabstandhaltergruppe und jedes Acc eine Elektronenakzeptorgruppe darstellt.

4. Chromophor nach Anspruch 1, wobei jedes R unabhängig eine funktionelle Gruppe darstellt, die aus der Gruppe ausgewählt ist, die aus Mesityl, 2-Ethylhexyl und einer Struktur der allgemeinen Formel besteht, wobei die Struktur am an das Chromophor gebunden ist

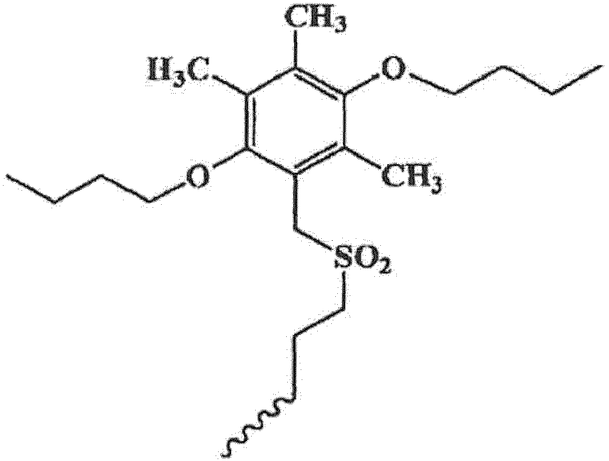

5. Chromophor nach Anspruch 1, wobei jedes R eine Mesitylgruppe darstellt.

6. Chromophor nach Anspruch 1, wobei jedes R eine 2-Ethylhexylgruppe darstellt.

7. Chromophor nach Anspruch 1, wobei jedes R eine Cyclohexylgruppe darstellt.

8. Nichtlineare optische Chromophor-Zusammensetzung, umfassend ein Gemisch aus zwei oder mehr stabilisierten Radikalen, die aus der Gruppe ausgewählt sind, die aus ausgewählten Radikalen der allgemeinen Formel (I'), Nitro-Radikalen der allgemeinen Formel (Ia) und Nitroxyl-Radikalen der allgemeinen Formel (Ib) besteht: 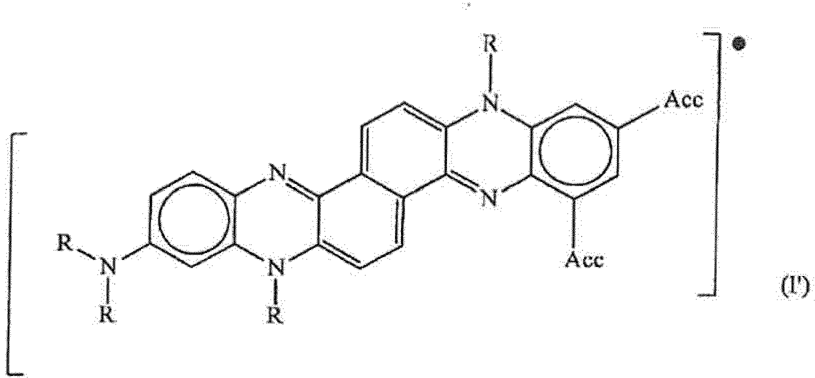 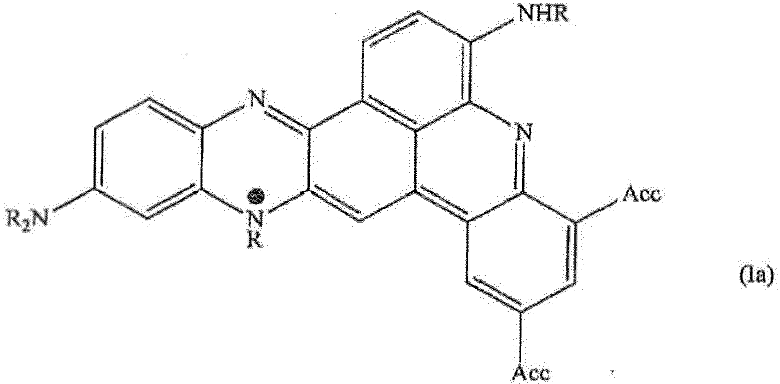 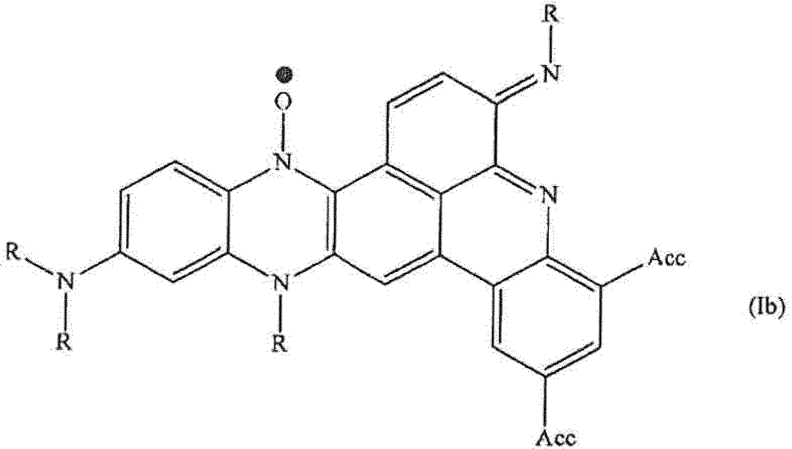 worin jedes R unabhängig eine Seitenabstandhaltergruppe und jedes Acc eine Elektronenakzeptorgruppe darstellt.

## Revendications

1. Chromophore optique non linéaire comprenant un radical stabilisé de la formule générale 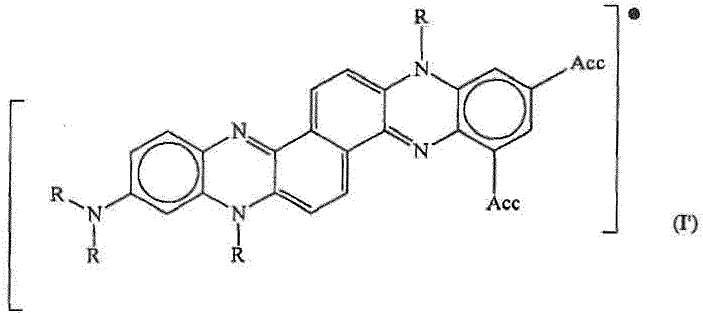 sachant que chaque R représente indépendamment un groupe espaceur pendant et chaque Acc représente un groupe accepteur d'électrons.

2. Chromophore optique non linéaire comprenant un radical stabilisé de la formule générale 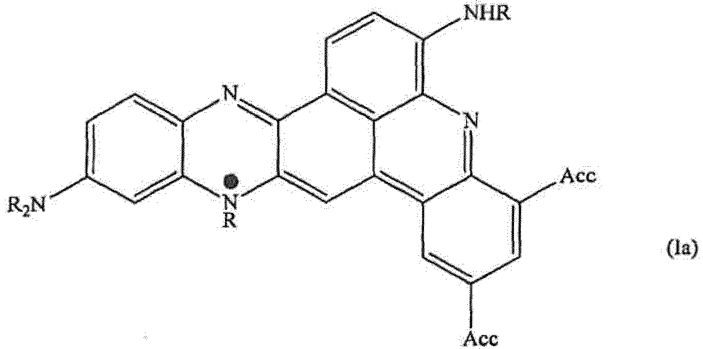 sachant que chaque R représente indépendamment un groupe espaceur pendant et chaque Acc représente un groupe accepteur d'électrons.

3. Chromophore optique non linéaire comprenant un radical stabilisé de la formule générale 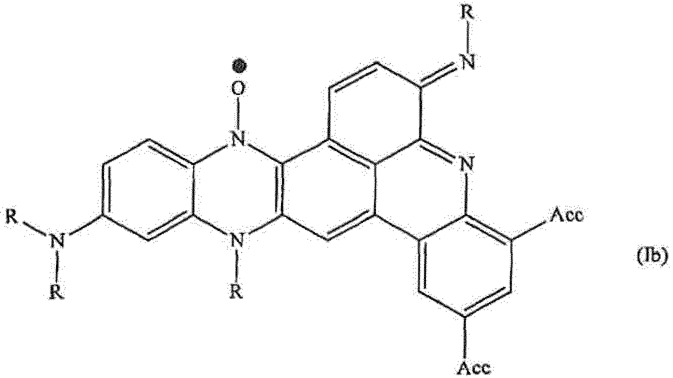 sachant que chaque R représente indépendamment un groupe espaceur pendant et chaque Acc représente un groupe accepteur d'électrons.

4. Le chromophore selon la revendication 1, sachant que chaque R représente indépendamment un groupe fonctionnelsélectionné dans le groupe constitué par le mésityle, le 2-éthylhexyle et une structure de la formule générale, sachant que la structure est liée au chromophore au niveau du

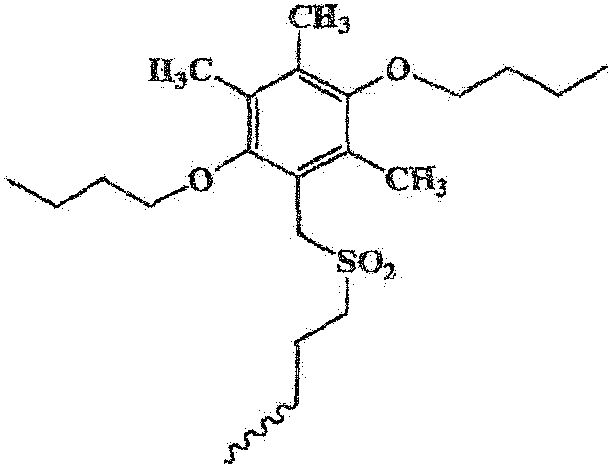

5. Le chromophore selon la revendication 1, sachant que chaque R représente un groupe mésityle.

6. Le chromophore selon la revendication 1, sachant que chaque R représente un groupe 2-éthylhexyle.

7. Le chromophore selon la revendication 1, sachant que chaque R représente un groupe cyclohexyle.

8. Composition de chromophore optique non linéaire comprenant un mélange de deux radicaux stabilisés ou plus sélectionnés dans le groupe constitué par des radicaux de la formule générale (I'), des nitroradicaux de la formule générale (la) et des radicaux de nitroxyle de la formule générale (Ib) : 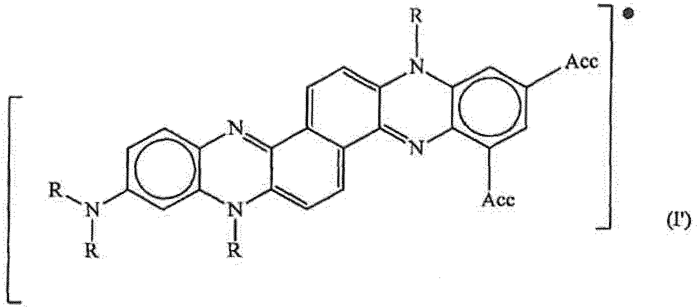 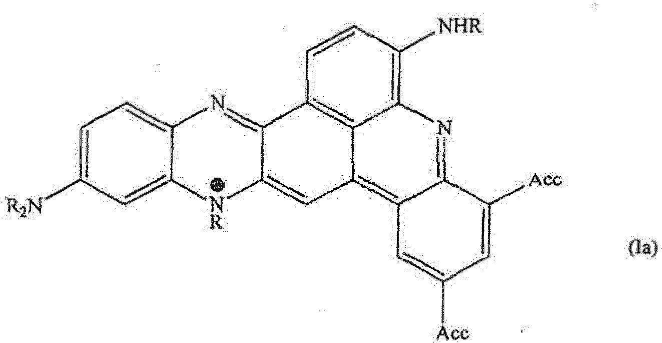 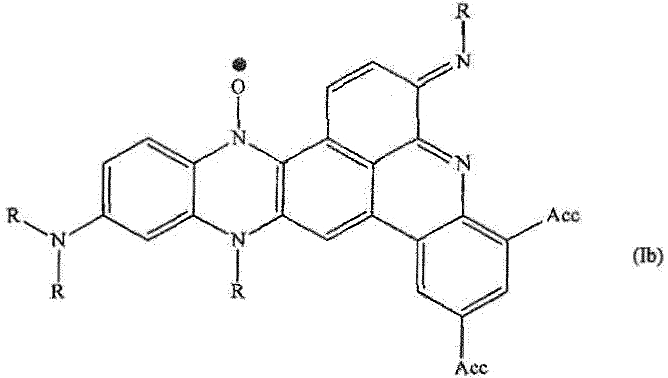 sachant que chaque R représente indépendamment un groupe espaceur pendant et chaque Acc représente un groupe accepteur d'électrons.
